# EUROPÄISCHE PATENTSCHRIFT

(11) **EP 4 699 588 B1**
(45) Veröffentlichungstag und Bekanntmachung des Hinweises auf die Patenterteilung: **18.11.2026**
(21) Anmeldenummer: 25194140.7
(22) Anmeldetag: 05.08.2025
(51) Int. Cl.: A61F 13/62, A44B 18/00

(54) **VERBINDUNGSSYSTEM UND VERFAHREN ZUM LÖSEN EINER HAFTVERBINDUNG**
CONNECTION SYSTEM AND METHOD FOR RELEASING AN ADHESIVE CONNECTION
SYSTÈME DE CONNEXION ET PROCÉDÉ DE LIBÉRATION D'UNE CONNEXION ADHÉSIVE

(30) Priorität: 13.08.2024 DE 102024123073
(43) Veröffentlichungstag der Anmeldung: 25.02.2026
(73) Patentinhaber: Gottlieb Binder GmbH & Co. KG, 71088 Holzgerlingen (DE)
(72) Erfinder: HEEPE, Lars, 72072 Tübingen (DE); DROTLEF, Dirk-Michael, 75233 Tiefenbronn (DE)
(74) Vertreter: Kohler Schmid Möbus Patentanwälte

(56) Entgegenhaltungen:
- DE-T2- 69 624 784

## Beschreibung

### Hintergrund der Erfindung

Die Erfindung betrifft ein Verbindungssystem mit einem ersten Haftmittel, welches eine Mehrzahl von Haftelementen aufweist, und einem Trägerelement, wobei das erste Haftmittel ein erstes freies Ende aufweist und an einem, vom ersten freien Ende beabstandeten, Anbindungsbereich schwenkbar an dem Trägerelement angeordnet oder ausgebildet ist, wobei ein erster schwenkbarer Teil des ersten Haftmittels gegenüber dem Trägerelement beweglich ist und wobei der erste schwenkbare Teil eine Länge L vom ersten freien Ende des ersten Haftmittels bis zum Beginn des Anbindungsbereichs aufweist. Die Erfindung betrifft auch einen Hygieneartikel und ein Verfahren zum Lösen einer Haftverbindung.

Verbindungssysteme zur Herstellung von Haftverbindungen, insbesondere manuell wiederlösbarer Haftverbindungen, weisen vielfach flächige Haftmittel mit einer Vielzahl von Haftelementen auf, welche von einem Nutzer - auch einem Nutzer im Kleinkindalter - intuitiv gehandhabt werden können. Beim Lösen einer derartigen Haftverbindung wird in der Regel ein freies Ende eines der Haftmittel gegriffen und durch eine Schälbewegung von dem anderen Haftmittel getrennt. Die Methode des Abschälens wird der Nutzer intuitiv wählen, da in diesem Fall die aufzuwendende Kraft maßgeblich von der Breite einer Schälfront bestimmt wird. Der Betrag der aufzuwendenden Kraft ist dabei beträchtlich geringer als wenn der Nutzer versuchen würde, die Haftmittel mittels einer Schubbewegung oder durch ein senkrechtes Abheben voneinander zu trennen, wobei die aufzuwendende Kraft maßgeblich von der Fläche der aneinander angehafteten Bereiche der Haftmittel bestimmt wird. Daraus folgt, dass das Lösen einer entsprechenden Haftverbindung für gewöhnlich durch die Disposition eines freien Endes eines der Haftmittel oder eines, mit einem der Haftmittel verbundenen, Trägerelements in Richtung der (noch) aneinander angehafteten Bereiche der Haftmittel erfolgt.

Die gattungsgemäße EP 2 244 599 B1 beschreibt eine lösbare Befestigungsanordnung, beispielsweise in Form eines Klettverschlusses, wobei innerhalb eines Haftmittels eine flexible Klappe ausgebildet ist. Die flexible Klappe trägt, wie auch die die Klappe umgebenden Bereiche, Haftelemente und kann aus der Ebene des Haftmittels schwenken. Bei einem Trennen der Haftverbindung durch Abschälen, wobei sich eine Schälfront in einer Richtung von einem freien Ende der Klappe zur einem angebundenen Ende derselben bewegt, verbleibt die Klappe, während sie von der Schälfront passiert wird, zunächst angehaftet. Anschließend wird aufgrund des angebundenen Endes eine Zugkraft auf die Klappe ausgeübt, wobei diese jedoch nicht vom korrespondierenden Haftmittel abgeschält, sondern abgezogen wird. Die Eigenschaften eines resultierenden Schälwiderstandes der gesamten Befestigungsanordnung, und damit der zum Lösen einer entsprechenden Haftverbindung aufzuwendenden Kraft, können somit anhand der Auslegung der Klappe angepasst werden. Insbesondere kann der resultierende Schälwiderstand erhöht werden und/oder es können diskrete Diskontinuitäten des Schälwiderstandes in bestimmten Bereichen vorgesehen werden.

US 8,685,194 B2 beschreibt ein Verfahren zur Herstellung von Haftverschlüssen. In einer Ausführungsvariante umfasst das Verfahren die Ausbildung diskreter Anti-Schäl-Klappen (anti-peel flaps) in einem Haftmittel mittels einer Vielzahl von Schlitzen. Die Klappen sind beispielsweise gegenüber einer Ebene eines Trägermaterials schwenkbar und eignen sich zum Hervorrufen des oben beschriebenen Effekts der Umwandlung eines Schälprinzips in ein Schubprinzip während eines Ablösevorgangs des Haftmittels von einem entsprechenden Gegenstück. In einigen Fällen sind die Schlitze an freien Kanten des Haftmittels ausgebildet.

Weitere Stand der Technik ist aus DE69624894T2 bekannt.

### Aufgabe der Erfindung

Es ist Aufgabe der Erfindung, ein Verbindungssystem zu schaffen, welches unter Ausnutzung des bekannten Prinzips der Abhängigkeit einer zum Lösen einer Haftverbindung aufzuwendenden Kraft von einem Schälwinkel bzw. einer Abzugsrichtung erweiterte Anwendungsmöglichkeiten gegenüber dem Stand der Technik bietet. Es ist weiterhin Aufgabe der Erfindung, einen Hygieneartikel und ein Verfahren zum Lösen einer Haftverbindung bereitzustellen.

### Beschreibung der Erfindung

Diese Aufgabe wird gelöst durch ein Verbindungssystem nach Anspruch 1, einen Hygieneartikel nach Anspruch 12 und ein Verfahren nach Anspruch 13. Die abhängigen Ansprüche geben bevorzugte Weiterbildungen wieder.

Befindet sich ein Verbindungssystem der eingangs genannten Art in einem Zustand, in dem ein erster schwenkbarer Teil eines ersten Haftmittels gegenüber einem Anbindungsbereich des ersten Haftmittels an einem Trägerelement nicht abgewinkelt ist, so sind freie Kanten des ersten schwenkbaren Teils nicht unmittelbar von Haftelementen des ersten Haftmittels umgeben.

Mit anderen Worten bedeutet dies, dass zu den freien Kanten des ersten schwenkbaren Teils des ersten Haftmittels auch in einem Zustand, in dem dieses nicht gegenüber einer Hauptebene des flächigen Trägerelements und/oder eines an dem Trägerelement angebundenen Teils des ersten Haftmittels verschwenkt ist, in einer Hauptebene des ersten schwenkbaren Teils nicht zu jeweils beiden Seiten entlang der Ausdehnung der freien Kanten weitere Haftelemente des ersten Haftmittels direkt benachbart sind. Somit sind zumindest zu einer Seite einer jeweiligen Kante, innerhalb eines Abstandes, der kleiner oder gleich dem mittleren Abstand einer Mehrzahl der Haftelemente des ersten Haftmittels untereinander ist, keine Haftelemente vorhanden.

Vorzugsweise weist der erste schwenkbare Teil eine zumindest annähernd rechteckige Form auf, wobei drei der Seiten des annähernden Rechtecks freie Kanten bilden, wobei die Kante, welche dem Anbindungsbereich gegenüberliegt, zugleich ein erstes freies Ende des ersten Haftmittels darstellt. Dennoch ist auch ein anderweitig geformter erster schwenkbarer Teil denkbar. Beispielsweise kann das erste freie Ende abgerundet sein, wodurch zwar eine einzige, gekrümmte freie Kante bedingt sein kann, die allerdings über den Verlauf ihrer Ausdehnung die freien Kanten der annähernden Rechteckanordnung jeweils abbildet und daher im vorliegenden Kontext als ,mehrere freie Kanten mit fließendem Übergang' betrachtet wird. Weiterhin kann das erste freie Ende ausgefranst sein oder mehrere Spitzen aufweisen, woraus eine Vielzahl freier Kanten resultiert.

Die maximale Ausdehnung einer Strecke von dem ersten freien Ende des ersten Haftmittels, in dessen Längsrichtung, auf den Beginn des Anbindungsbereiches bestimmt eine Länge L des ersten schwenkbaren Teils des ersten Haftmittels. Die maximale Ausdehnung des ersten Haftmittels senkrecht zur Länge L bestimmt dessen Breite B. Bevorzugt weist das Trägerelement die gleiche Breite oder eine größere Breite wie das erste Haftmittel auf.

Das erste Haftmittel weist die Mehrzahl der Haftelemente auf, wobei zumindest eines der Haftelemente, vorzugsweise jedoch mehrere der Haftelemente, am ersten schwenkbaren Teil angeordnet oder ausgebildet ist/sind.

Das Trägerelement ist vorzugsweise als flexibles Band ausgebildet. Insbesondere weist das Trägerelement seinerseits ebenfalls ein freies Ende auf. Ist das erste Haftmittel an einem zweiten Haftmittel angebracht, kann über ein Angreifen am Trägerelement das Lösen der Haftverbindung eingeleitet werden. Ein Nutzer wird dabei intuitiv versuchen, die Haftverbindung durch Abschälen des Trägerelements zu lösen. Bis zum Erreichen des Anbindungsbereichs wird bei diesem Abschälversuch allerdings zunächst nur das Trägerelement bewegt. Wird das Abschälen des Trägerelements fortgesetzt, wird, nachdem eine imaginäre Schälfront den Anbindungsbereich erreicht hat, eine Zugkraft auf das erste Haftmittel ausgeübt. Die zum Lösen der Haftverbindung notwendige Kraft ist dabei beträchtlich größer als diejenige, welche für ein tatsächliches Abschälen des ersten Haftmittels nötig wäre.

Zum Erreichen des tatsächlichen Abschälens muss ein Nutzer das freie Ende des Trägerelements entgegen der intuitiv gewählten Schälrichtung ziehen. Dabei wird die angelegte Kraft über eine freie Länge des Trägerelements zum Anbindungsbereich geleitet, wobei eine Schälfront an diesem beginnt. Durch den Gegensatz der vorgesehenen Methode zum Trennen der Haftmittel und der intuitiv naheliegenden Methode, weist das Verbindungssystem ein Sicherheitsmerkmal auf. Somit kann ein leichtfertiges Lösen der Haftverbindung, insbesondere auch durch Kleinkinder, verhindert werden. Weiterhin ist eine Realisierung des Sicherheitsmerkmals produktionstechnisch einfach möglich.

Der erste schwenkbare Teil weist insbesondere eine größere Anzahl von Haftelementen auf als der Anbindungsbereich. Vorzugsweise weist der erste schwenkbare Teil mehr als 50 %, bevorzugt mehr als 70 %, besonders bevorzugt mehr als 80 % der Haftelemente des ersten Haftmittels auf.

Bei einer bevorzugten Ausführung des Verbindungssystems ist die Gesamtheit der Haftelemente des ersten Haftmittels am ersten schwenkbaren Teil des ersten Haftmittels und/oder an einem zweiten schwenkbaren Teil des ersten Haftmittels angeordnet oder ausgebildet. Der zweite schwenkbare Teil kommt im Rahmen einer später erläuterten Weiterbildung der Erfindung zum Einsatz. Falls das erste Haftmittel den zweiten schwenkbaren Teil aufweist, weisen der erste und der zweite schwenkbare Teil gemeinsam vorzugsweise mehr als 67 %, besonders bevorzugt mehr als 80 % der Haftelemente des ersten Haftmittels auf.

Um einer am Übergang zwischen Anbindungsbereich und erstem schwenkbaren Teil beginnenden Schälfront keinen zusätzlichen Widerstand entgegenzusetzen, kann es vorteilhaft sein, wenn der Anbindungsbereich nicht mit Haftelementen belegt ist.

Vorzugsweise ist der erste schwenkbare Teil des ersten Haftmittels ein flächiger Streifen, wobei das Verhältnis der Länge L zu der Breite B im Bereich zwischen 0,1:1 und 4:1, insbesondere zwischen 0,2:1 und 2:1, liegt.

Die Anbindung des ersten Haftmittels an das Trägerelement kann insbesondere mittels eines geeigneten Klebstoffes oder eines thermischen Fügeverfahrens erfolgen.

Die Haftelemente des ersten Haftmittels können insbesondere Hakenelemente, pilzförmige Haftelemente, hyperboloidförmige Haftelemente oder palmenförmige Haftelemente eines Klett- oder Clipsverschlusses sein. Ggf. können die Haftelmente jeweils mehreren der vorgenannten Formen zugewiesen werden. Gleichermaßen ist denkbar, dass das erste Haftmittel Schlaufen und/oder Fasern eines Flauschbandes, Veloursbandes, Vliesstoffes oder eines Gewebes, Gewirkes oder Gestricks aufweist.

In einer weiteren Ausführungsvariante kann das erste Haftmittel Nano- und/oder Mikrostrukturen aufweisen. Insbesondere kann das erste Haftmittel dabei Vorsprünge mit einer Flächendichte von 10.000 Vorsprüngen bis 100.000 Vorsprüngen pro cm² aufweisen. Derartige Flächendichten bewirken eine starke Adhäsionskraft an einer hinreichend glatten Gegenfläche. Die Vorsprünge können eine Höhe von mindestens 20 µm, von mindestens 30 µm, oder von mindestens 40 µm aufweisen. Die Vorsprünge weisen in der Regel eine Höhe auf, die nicht größer ist als 800 µm, insbesondere nicht größer ist als 150 µm. Das Aspektverhältnis der Vorsprünge, d.h. das Verhältnis der Höhe zur Breite der Vorsprünge, liegt bevorzugt zwischen 0,1 und 3,0.

Bevorzugt weist/weisen das erste Haftmittel und/oder das Trägerelement Kunststoff auf. Geeignet sind dabei beispielsweise Polypropylen, Polyamid, Polyethylen, Polyurethan, Polysiloxan und/oder ein thermoplastisches Elastomer. Besonders bevorzugt weist/weisen das Trägerelement und/oder das erste Haftmittel ein biobasiertes und/oder biologisch abbaubares Kunststoffmaterial auf. Hier eignen sich beispielsweise Polycaprolacton, Polybutyrat, Polybutylensuccinat, Celluloseacetat, biobasiertes Polyethylen, Polyethylenfuranoat, biobasiertes Polyamid, biobasiertes Polyethylenterephthalat, biobasiertes Polypropylen, Polylactid, stärkebasierter Kunststoff und/oder Polyhydroxalkanoat.

Die maximale Ausdehnung einer Strecke von dem freien Ende des Trägerelements, entlang dessen Längsrichtung, auf den Beginn des Anbindungsbereiches bestimmt die freie Länge T des Trägerelements. Die Länge T kann bevorzugt bis 500 % der Länge L des ersten schwenkbaren Teils des ersten Haftmittels betragen. Falls die Länge L größer als die Länge T sein soll, kann alternativ die Länge L bis 500 % der Länge T betragen. Vorzugsweise liegt die Länge T in einem Bereich von ± 20 % der Länge L des ersten schwenkbaren Teils des ersten Haftmittels.

Bei einer Weiterbildung der Erfindung weist das erste Haftmittel ein, dem ersten freien Ende gegenüberliegendes, zweites freies Ende auf, wobei sich der Anbindungsbereich zwischen dem ersten freien Ende und dem zweiten freien Ende befindet. Dabei weist das erste Haftmittel den erwähnten zweiten schwenkbaren Teil auf. Im Rahmen der Weiterbildung ist ein einfaches Abschälen des angehafteten ersten Haftmittels entlang der Länge L mithilfe des Trägerelements nicht mehr möglich, da eine Schälfront vom Anbindungsbereich aus in zwei gegenläufige Richtungen laufen müsste. So kann im Rahmen der genannten Weiterbildung eine besonders schwer zu lösende Haftverbindung geschaffen werden. Diese kann in vorteilhafter Weise insbesondere durch ein Querabziehen (entlang der Breite B) gelöst werden, wobei auch in diesem Fall ein verhältnismäßig hoher Kraftaufwand notwendig ist, da beim Ziehen am Trägerelement in Querrichtung der Kraftangriff nicht gänzlich zur Ausbildung einer Schälfront geeignet ist.

Der gleiche Effekt kann über die Anordnung oder Ausbildung eines weiteren ersten Haftmittels an dem Trägerelement erreicht werden, wobei das weitere erste Haftmittel in einer Ebene des Trägerelements gegenüber dem ersten Haftmittel um 180° gedreht ist, wobei der Anbindungsbereich des ersten Haftmittels und ein weiterer Anbindungsbereich des weiteren ersten Haftmittels einander näher liegen als die jeweiligen ersten freien Enden der beiden ersten Haftmittel.

Die Länge des Anbindungsbereichs und ggf. des weiteren Anbindungsbereichs liegt bevorzugt in einem Bereich von 0,1 mm bis 10 mm. Falls der weitere Anbindungsbereich vorhanden ist, liegt der Abstand zwischen den Anbindungsbereichen bevorzugt im Bereich von 0,1 mm bis 100 mm.

Das Verbindungssystem weist weiterhin vorzugsweise das zweite Haftmittel auf, wobei das zweite Haftmittel zum Herstellen einer Haftverbindung mit dem ersten Haftmittel und/oder dem weiteren ersten Haftmittel geeignet ist. Dazu kann es insbesondere Hakenelemente, pilzförmige Haftelemente, hyperboloidförmige Haftelemente oder palmenförmige Haftelemente, oder Fasern und/oder Schlaufen eines Flauschbandes, Veloursbandes, Vliesstoffes oder eines Gewebes, Gewirkes oder Gestricks aufweisen. In dem Fall, dass das erste Haftmittel und/oder das weitere erste Haftmittel Nano- und/oder Mikrostrukturen aufweist, kann das zweite Haftmittel bevorzugt eine zum Zusammenwirken mit dem ersten Haftmittel hinreichend glatte Fläche, beispielsweise in Form einer Folie, aufweisen.

Der Verschluss eines Hygieneartikels, insbesondere einer Windel, lässt sich in besonders vorteilhafter Weise mittels des Verbindungssystems realisieren. Ein dem freien Ende des Trägerelements gegenüberliegendes Ende des Trägerelements ist dabei an den Hygieneartikel angebunden. Eine Anordnung des Trägerelements an dem Hygieneartikel kann vorzugsweise wiederum mittels eines Klebstoffes oder eines thermischen Fügeverfahrens realisiert sein. Insbesondere kann das Zusammenfügen des Hygieneartikels, des Trägerelements und des ersten Haftmittels (und ggf. des weiteren ersten Haftmittels) in einem Produktionsschritt erfolgen. Ebenfalls ist denkbar, dass das Trägerelement als integraler Teil des Hygieneartikels ausgebildet ist, wobei das angebundene Ende des Trägerelements durch den Übergang des Hauptkörpers des Hygieneartikels zu dem Trägerelement markiert wird. Weiter alternativ kann das Trägerelement an den Hauptkörper des Hygieneartikels angenäht werden.

Das Trägerelement bildet in Verbindung mit dem ersten Haftmittel (und ggf. dem weiteren ersten Haftmittel) eine Verschlusslasche des Hygieneartikels. Vorzugsweise weist der Hygieneartikel weiterhin das zweite Haftmittel auf.

Die Verschlusslasche kann unmittelbar an einem Körper des Hygieneartikels angeordnet sein. Insbesondere kann die Verschlusslasche unmittelbar an einem Windelkörper der Windel angeordnet sein. Alternativ dazu kann die Verschlusslasche über eine weitere Lage, insbesondere in Form eines sogenannten Ohrs, d.h. eine ohrförmig ausgebildete Lage, am Körper des Hygieneartikels angeordnet sein. Beispielsweise kann die Verschlusslasche über ein Windelohr am Windelkörper angeordnet sein.

Die Aufgabe wird weiterhin gelöst durch ein Verfahren zum Lösen einer Haftverbindung eines Verbindungssystems, welches das erste Haftmittel (und ggf. das weitere erste Haftmittel) und das zweite Haftmittel aufweist. Das Verfahren umfasst die Disposition des freien Endes des Trägerelements in eine Richtung von dem angebundenen Ende des Trägerelements hin zu dem freien Ende des Trägerelements, d.h. ein Ziehen des Trägerelements weg vom angebundenen Ende, um so ein Abschälen des ersten Haftmittels vom zweiten Haftmittel zu erreichen. Dies entspricht dem oben beschriebenen kontraintuitiven Vorgehen beim Lösen einer gattungsgemäßen Haftverbindung. Insbesondere falls das Abschälen durch eine entsprechende Ausbildung des ersten Haftmittels und/oder des weiteren ersten Haftmittels in der genannten Richtung gänzlich unterbunden/erschwert ist, sieht das Verfahren das Ziehen des Trägerelements quer zur genannten Richtung vor. Dadurch wird das erste Haftmittel und/oder ggf. das weitere erste Haftmittel vom zweiten Haftmittel getrennt.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Kurzbeschreibung der Zeichnung

Es zeigen:
- Fig. 1a: eine perspektivische Ansicht eines ersten Haftmittels und eines Ausschnitts eines Trägerelements;
- Fig. 1b: eine weitere perspektivische Ansicht des ersten Haftmittels und eines Ausschnitts des Trägerelements aus Fig. 1a;
- Fig. 2a: einen Ausschnitt eines Verbindungssystems, welches das erste Haftmittel, das Trägerelement und ein zweites Haftmittel aufweist, in einer Seitenansicht;
- Fig. 2b: einen Graphen, der schematisch den Betrag einer Kraft, welche zum Lösen des Verbindungssystems aus Fig. 2a aufgewendet werden muss, bezogen auf einen Schälwinkel darstellt;
- Fig. 3: ausschnittsweise eine weitere Ausführungsvariante eines Verbindungssystems in einer Seitenansicht;
- Fig. 4: ausschnittsweise eine weitere Ausführungsvariante eines Verbindungssystems in einer Seitenansicht;
- Fig. 5: ausschnittsweise eine weitere Ausführungsvariante eines Verbindungssystems in einer Seitenansicht;
- Fig. 6a: eine perspektivische Ansicht einer weiteren Ausführungsvariante eines ersten Haftmittels und eines Ausschnitts eines Trägerelements;
- Fig. 6b: eine weitere perspektivische Ansicht des ersten Haftmittels und des Trägerelements aus Fig. 6a;
- Fig. 6c: eine ausschnittsweise Seitenansicht eines Verbindungssystems mit dem ersten Haftmittel und dem Trägerelement aus den Fign. 6a und 6b und einem zweiten Haftmittel;
- Fig. 7: eine perspektivische Ansicht eines ersten Haftmittels und eines weiteren ersten Haftmittels sowie eines Ausschnitts eines Trägerelements;
- Fig. 8: einen Hygieneartikel in Form einer Windel mit einem Verbindungssystem;
- Fig. 9: ein Diagramm, welches ein Verfahren zum Lösen einer Haftverbindung abbildet.

### Detaillierte Beschreibung der Erfindung und Zeichnung

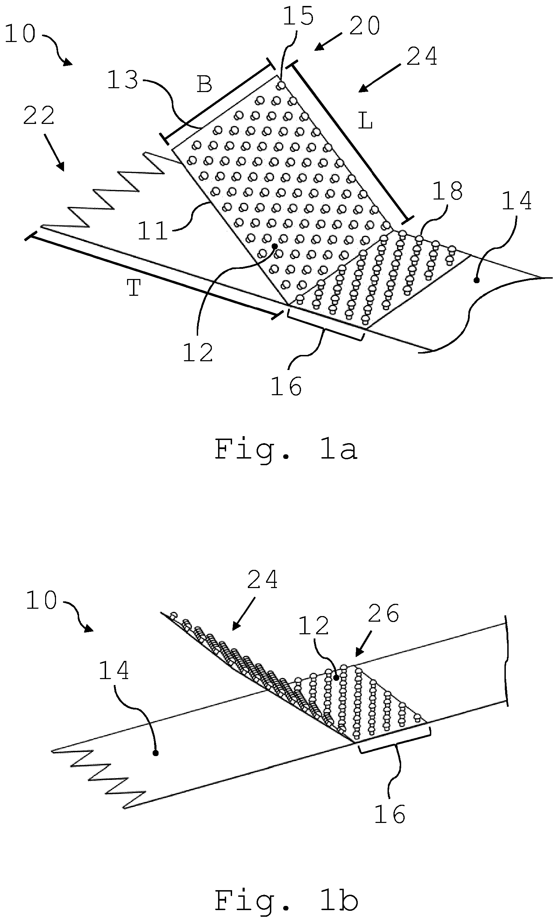

**Fig. 1a** zeigt eine perspektivische Ansicht eines Verbindungssystems **10** mit einem ersten Haftmittel **12,** welches drei freie Kanten **11, 13,** und **15** aufweist. Die freie Kante 13 bildet zugleich ein erstes freies Ende **20** des ersten Haftmittels 12. Das erste Haftmittel 12 ist in einem Anbindungsbereich **16** an einem Trägerelement **14** angeordnet. Ein erster schwenkbarer Teil **24** des ersten Haftmittels 12 schließt sich an den Anbindungsbereich 16 an.

Der erste schwenkbare Teil 24 kann gegenüber dem Trägerelement 14 und dem Anbindungsbereich 16 verschwenkt werden und weist eine Länge **L** von der freien Kante 13 bis zum Beginn des Anbindungsbereichs 16 sowie eine Breite **B** von der freien Kante 11 zur freien Kante 15 auf. Das Trägerelement 14 ist ausschnittsweise dargestellt. Es weist seinerseits ein freies Ende **22** auf, wobei eine Länge **T** die maximale Länge des Trägerelements 14 von dem freien Ende 22 bis hin zum Beginn des Anbindungsbereichs 16 bezeichnet. Die Länge T ist in diesem Fall größer als die Länge L.

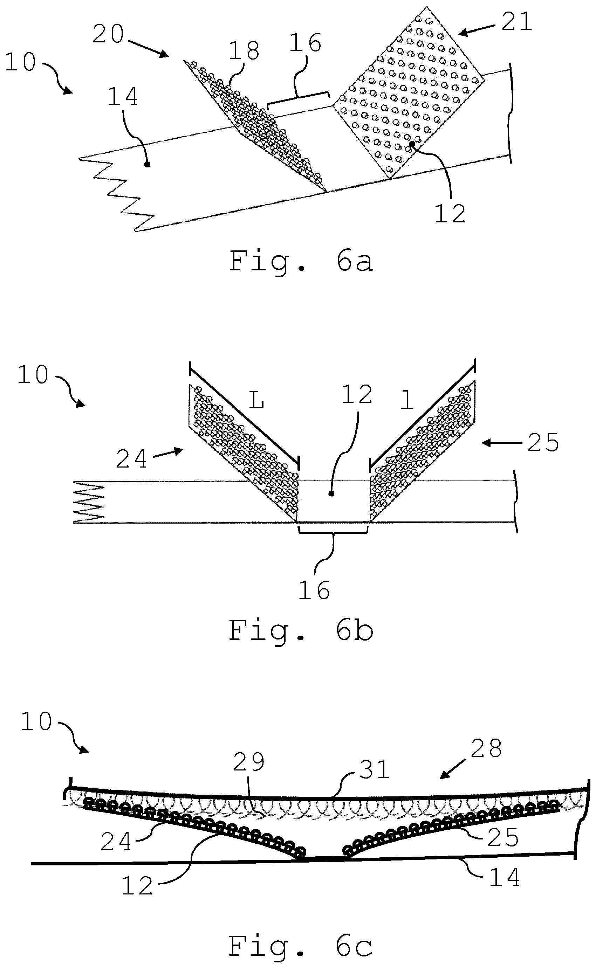

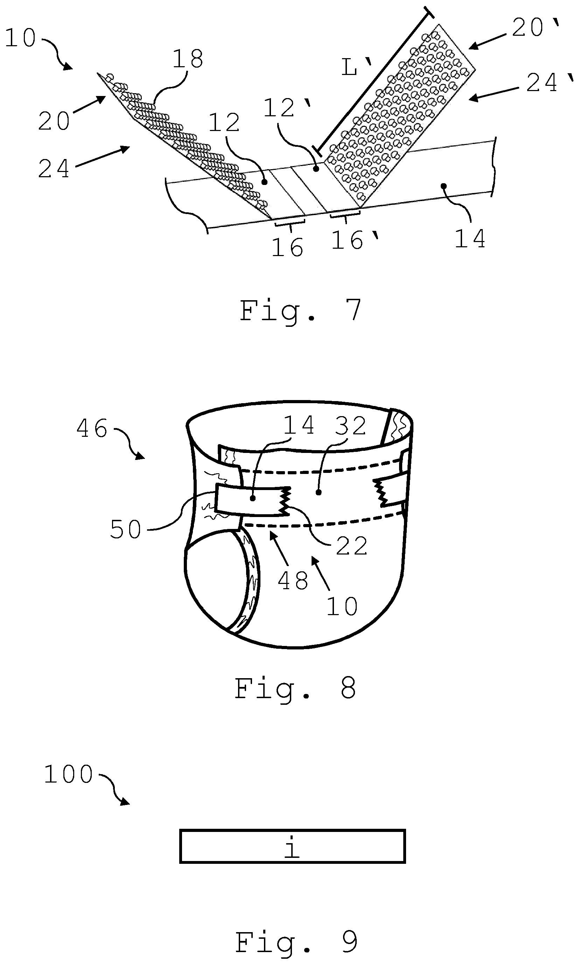

Das erste Haftmittel 12 trägt in der dargestellten Ausführungsvariante eine Vielzahl von pilzförmigen Haftelementen **18.** Die Haftelemente 18 besetzen sowohl den ersten schwenkbaren Teil 24 als auch den Anbindungsbereich 16. Dabei weist der erste schwenkbare Teil 24 eine größere Anzahl der pilzförmigen Haftelemente 18 auf als der Anbindungsbereich 16. Im dargestellten Ausführungsbeispiel befinden sich etwa 70 % der vorhandenen Haftelemente 18 am ersten schwenkbaren Teil 24. In weiteren Ausführungsvarianten (vergleiche Fign. 6a und 7) weist der Anbindungsbereich 16 keine Haftelemente 18 auf. Ebenso ist denkbar, dass der erste schwenkbare Teil 24 derart am Trägerelement 14 ausgebildet ist, dass der Anbindungsbereich 16 nicht als separater Bereich bzw. als separate Lage auf dem Trägerelement 14 wahrnehmbar ist, d.h. Teil des Materials der Trägerelements 14 ist (nicht dargestellt). Nicht mit dem ersten Haftmittel 12 verbundene Bereiche des Trägerelements 14 können bei einer flexiblen Ausführung des Trägerelements 14 gleichermaßen gegenüber dem ersten Haftmittel 12 verschwenkt werden.

**Fig. 1b** zeigt eine weitere perspektivische Ansicht des Verbindungssystems 10 aus Fig. 1a. Das Trägerelement 14 ist ausschnittsweise dargestellt und der erste schwenkbare Teil 24 des ersten Haftmittels 12 ist gegenüber dem Trägerelement 14 sowie dem Anbindungsbereich 16 verschwenkt. Das erste Haftmittel ist im dargestellten Ausführungsbeispiel mittels eines Klebstoffs **26** an dem Trägerelement 14 angeordnet.

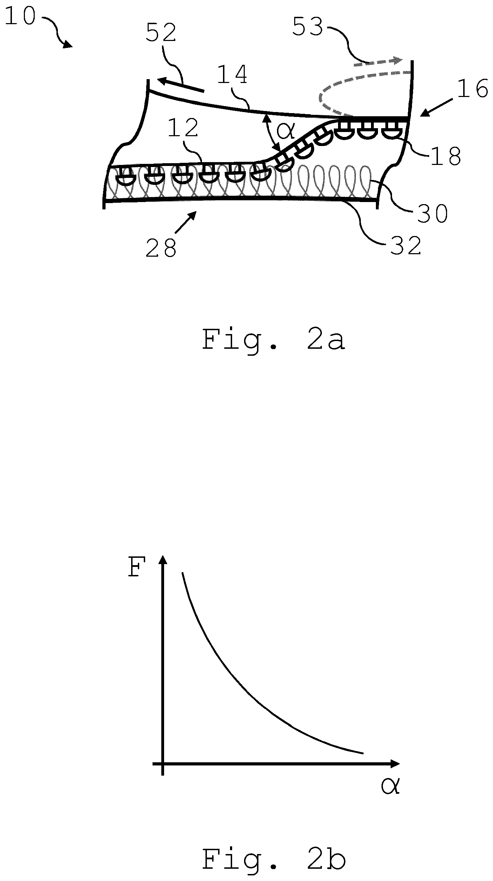

**Fig. 2a** zeigt ausschnittsweise eine weitere Variante eines Verbindungssystems 10 in einer Seitenansicht, wobei das Verbindungssystem 10 ein erstes Haftmittel 12 und ein Trägerelement 14 aufweist, welche analog zu der Ausführung in den Fign. 1a und 1b gestaltet sind. Weiterhin weist das Verbindungssystem 10 ein zweites Haftmittel **28,** hier in Form eines Flauschbandes **32,** auf. Schlaufen **30** des Flauschbandes 32 sind teilweise mit pilzförmigen Haftelementen 18 des ersten Haftmittels 12 verhakt. In Fig. 2a ist schematisch ein Zustand während des Ablösens des ersten Haftmittels 12 vom zweiten Haftmittel 28 dargestellt. Das Trägerelement 14 wird dabei an seinem freien Ende 22 (siehe Fig. 1a) gegriffen, in Richtung des in der Darstellung noch angehafteten Bereichs gezogen und dabei gleichzeitig vom zweiten Haftmittel 28 abgehoben (entspricht in der Darstellung einer durch einen Pfeil **52** angedeuteten Zugrichtung).

Nachdem der Anbindungsbereich 16 vom zweiten Haftmittel 28 getrennt wurde, stellt sich dabei eine Schälfront mit einem Schälwinkel **a** ein (vergleiche dazu Fig. 2b). Bei einem hypothetischen Trennungsversuch durch ein intuitives Abschälen des Trägerelements 14 (entlang einer Zugrichtung **53)** würde das erste Haftmittel 12 nicht vom zweiten Haftmittel 28 abgeschält werden. Stattdessen müsste das erste Haftmittel 12 unter großem Kraftaufwand flächig vom zweiten Haftmittel 28 abgezogen werden.

**Fig. 2b** zeigt vereinfacht einen Zusammenhang zwischen dem Schälwinkel a in einem Bereich von 180° bis 90° und einer zum Ablösen des ersten Haftmittels 12 (siehe Fig. 2a) vom zweiten Haftmittel 28 (siehe Fig. 2a) initial aufzuwendenden Kraft **F.** Vorzugsweise liegt a zwischen 160° und 90°.

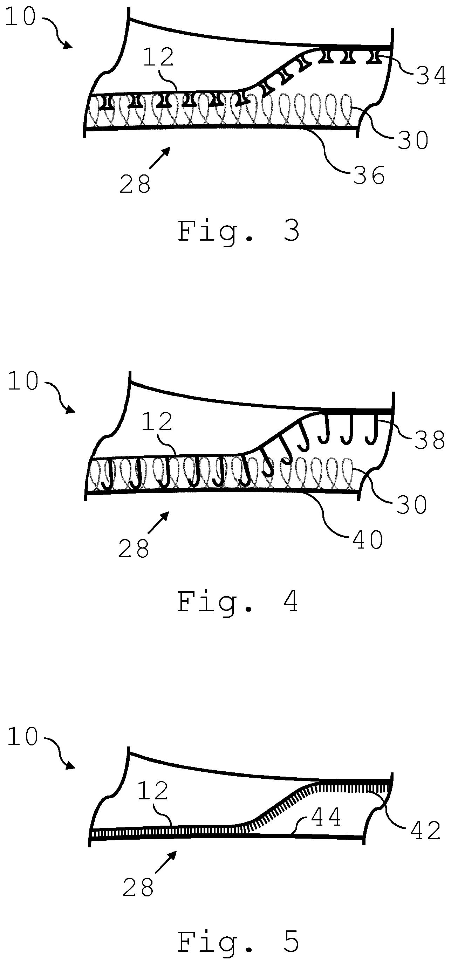

**Fig. 3, Fig. 4** und **Fig. 5** zeigen jeweils ausschnittsweise weitere Varianten eines Verbindungssystems 10. Die Darstellung umfasst in Analogie zu Fig. 2a jeweils einen Ausschnitt eines ersten Haftmittels 12 und eines zweiten Haftmittels 28, welche teilweise aneinander angehaftet sind. Die Verbindungssysteme 10 unterscheiden sich jeweils durch die Art der verwendeten Haftmittel 12, 28. In Fig. 3 weist ein erstes Haftmittel 12 palmenförmige Haftelemente **34** auf, während ein zweites Haftmittel 28 Schlaufen 30 eines Gewebes **36** aufweist. Die palmenförmigen Haftelemente 34 könnten im dargestellten Ausführungsbeispiel gleichermaßen als hyperboloidförmige Haftelemente 34 bezeichnet werden. In Fig. 4 weist ein erstes Haftelement 12 Hakenelemente **38** zum Zusammenwirken mit Schlaufen 30 eines Velourbandes **40** auf. In Fig. 5 hingegen weist ein erstes Haftmittel 12 Nano- und/oder Mikrostrukturen **42** auf, während ein zweites Haftmittel 28 eine glatte Fläche **44** aufweist.

**Fig. 6a** zeigt eine perspektivische Ansicht einer weiteren Variante eines Verbindungssystems 10, wobei ein erstes Haftmittel 12 ein erstes freies Ende 20 und ein zweites freies Ende **21** aufweist. Ein Trägerelement 14 ist wiederum ausschnittsweise dargestellt. Ein Anbindungsbereich 16 des ersten Haftmittels 12 befindet sich zwischen dem ersten freien Ende 20 und dem zweiten freien Ende 21. Der Anbindungsbereich 16 trägt in der dargestellten Ausführungsvariante keine der pilzförmigen Haftelemente 18.

**Fig. 6b** zeigt eine weitere perspektivische Ansicht des Verbindungssystems 10 aus Fig. 6a. Es wird deutlich, dass das erste Haftmittel 12 einen ersten schwenkbaren Teil 24 mit einer Länge L und einen zweiten schwenkbaren Teil **25** mit einer Länge I aufweist. Die Längen L und I können wie dargestellt den gleichen Betrag aufweisen. Dies ist jedoch nicht zwingend notwendig. Die Länge des Anbindungsbereichs 16 kann abhängig vom Anwendungsfall gewählt werden.

**Fig. 6c** zeigt ausschnittsweise ein Verbindungssystem 10 in einer Seitenansicht. Das Verbindungssystem 10 weist das in den Fign. 6a und 6b dargestellte erste Haftmittel 12 mit dem ersten schwenkbaren Teil 24 und dem zweiten schwenkbaren Teil 25 sowie das Trägerelement 14 auf. Zusätzlich weist das Verbindungssystem 10 ein zweites Haftmittel 28 auf, welches teilweise an dem ersten Haftmittel 12 angehaftet ist. Das zweite Haftmittel 28 weist Fasern **29** eines Vliesstoffes **31** auf.

**Fig. 7** zeigt ausschnittsweise eine perspektivische Ansicht einer weiteren Variante eines Verbindungssystems 10. Ein erstes Haftmittel 12 weist ein erstes freies Ende 20, einen Anbindungsbereich 16 und einen ersten schwenkbaren Teil 24 auf. Ein weiteres erstes Haftmittel **12'** ist gegenüber dem ersten Haftmittel 12 um 180° gedreht an einem Trägerelement 14 angeordnet. Das weitere erste Haftmittel 12' weist analog zum ersten Haftmittel 12 ein weiteres erstes freies Ende **20',** einen weiteren Anbindungsbereich **16'** sowie einen weiteren ersten schwenkbaren Teil **24'** auf. Mittels der dargestellten Anordnung wird das in den Fign. 6a und 6b dargestellte Verbindungssystem 10 funktionell äquivalent nachgebildet. Eine Länge **L'** des weiteren ersten schwenkbaren Teils 24' kann dabei wie dargestellt der Länge L (siehe Fig. 1a) des ersten schwenkbaren Teils 24 entsprechen oder sich von dieser unterscheiden. Ebenso ist denkbar, dass die beiden ersten Haftmittel 12, 12' sich in ihrer Breite oder hinsichtlich der Dimensionierung ihrer jeweiligen Anbindungsbereiche 16, 16' unterscheiden. Weiterhin ist denkbar, dass das erste Haftmittel 12 und das weitere erste Haftmittel 12' jeweils mit unterschiedlichen Haftelementen 18 (vergleiche Fign. 3, 4 und 5) besetzt sind.

**Fig. 8** zeigt einen Hygieneartikel **46,** wobei ein Verbindungssystem 10 in einer der vorgestellten Varianten eine Verschlusslasche **48** des Hygieneartikels 46 ausmacht. Ein erstes Haftmittel 12 (hier nicht sichtbar, siehe z. B. Fig. 1a) ist an einem Flauschband 32 angehaftet, welches wiederum an dem Hygieneartikel 46 angeordnet oder ausgebildet ist. Ein Trägerelement 14 des Verbindungssystems 10 weist ein an den Hygieneartikel 46 angebundenes Ende **50** sowie ein freies Ende 22 auf. Unter der Annahme, dass das Verbindungssystem 10 wie in Fig. 2a dargestellt ausgebildet ist, kann die Verschlusslasche 48 durch ein Ziehen am freien Ende 22 des Trägerelements 14 in einer Richtung von dessen angebundenem Ende 50 hin zum freien Ende 22 leicht gelöst werden. Bei einem intuitiven Abschälen des freien Endes 22 des Trägerelements 14 in Richtung des angebundenen Endes 50, ist hingegen ein Ablösen der Verschlusslasche 48 nur unter vergleichsweise hohem Kraftaufwand möglich.

**Fig. 9** zeigt ein Diagramm eines Verfahrens **100** zum Lösen eines Verbindungssystems 10 (siehe z.B. Fig. 8). Der Verfahrensschritt **i** beinhaltet das Ziehen eines Trägerelements 14 (siehe Fig. 8) in eine Richtung weg von einem angebundenen Ende 50 (siehe Fig. 8) des Trägerelements 14 oder, insbesondere in dem Fall, in dem ein zweites freies Ende 21 (siehe Fig. 6a) und/oder ein weiteres erstes freies Ende 20' (siehe Fig. 7) an einem ersten Haftmittel 12 (siehe z.B. Fig. 7) vorhanden ist, das Ziehen des Trägerelements 14 quer zu einer Richtung von dem angebundenen Ende 50 des Trägerelements 14 zu dem freien Ende 22 des Trägerelements 14. Dadurch erfolgt das Trennen des ersten Haftmittels 12 und/oder eines weiteren ersten Haftmittels 12' (siehe Fig. 7) von einem zweiten Haftmittel 28 (siehe z.B. Fig. 2a).

Unter Vornahme einer Zusammenschau der Figuren der Zeichnung betrifft die Erfindung ein Verbindungssystem 10 mit einem ersten Haftmittel 12, welches eine Mehrzahl von Haftelementen 18, 29, 30, 34, 38, 42 aufweist. Das erste Haftmittel 12 weist ein erstes freies Ende 20 und einen Anbindungsbereich 16 auf, wobei der Anbindungsbereich 16 an einem, vorzugsweise als flexiblem Band ausgebildeten, Trägerelement 14 angeordnet oder ausgebildet ist. Ein erster schwenkbarer Teil 24 des ersten Haftmittels 12 ist gegenüber dem Trägerelement 14 beweglich, insbesondere schwenkbar. Der erste schwenkbare Teil 24 weist freie Kanten 11, 13, 15, auf. In einem Zustand, in dem der erste schwenkbare Teil 24 nicht gegenüber dem Anbindungsbereich 16 verschwenkt ist, d.h. in dem beide in einer gemeinsamen Ebene liegen, sind die freien Kanten 11, 13, 15 in dieser Ebene nicht unmittelbar von Haftelementen 18, 29, 30, 34, 38, 42 des ersten Haftmittels 12 umgeben. Der erste schwenkbare Teil 24 weist insbesondere eine größere Anzahl von Haftelementen 18, 29, 30, 34, 38, 42 auf als der Anbindungsbereich 16. Die Erfindung betrifft auch ein Verfahren 100 zum Lösen eines derartigen Verbindungssystems 10, wobei das Verbindungssystem 10 ein zweites Haftmittel 28 aufweist.

### Bezugszeichenliste

- 10: Verbindungssystem
- 11: freie Kante
- 12: erstes Haftmittel
- 12**'**: weiteres erstes Haftmittel
- 13: freie Kante
- 14: Trägerelement
- 15: freie Kante
- 16: Anbindungsbereich
- 16': weiterer Anbindungsbereich
- 18: pilzförmiges Haftelement
- 20: erstes freies Ende
- 20': weiteres erstes freies Ende
- 21: zweites freies Ende
- 22: freies Ende des Trägerelements
- 24: erster schwenkbarer Teil
- 24': weiterer erster schwenkbarer Teil
- 25: zweiter schwenkbarer Teil
- 26: Klebstoff
- 28: zweites Haftmittel
- 29: Faser
- 30: Schlaufe
- 31: Vliesstoff
- 32: Flauschband
- 34: palmenförmiges/hyperboloidförmiges Haftelement
- 36: Gewebe
- 38: Hakenelement
- 40: Veloursband
- 42: Mikrostruktur
- 44: glatte Fläche
- 46: Hygieneartikel
- 48: Verschlusslasche
- 50: angebundenes Ende des Trägerelements
- 52: vorgesehene Zugrichtung
- 53: intuitive Zugrichtung
- 100: Verfahren zum Lösen eines Verbindungssystems
- L: Länge des ersten schwenkbaren Teils
- L': Länge des weiteren ersten schwenkbaren Teils
- I: Länge des zweiten schwenkbaren Teils
- B: Breite des ersten Haftmittels
- T: freie Länge des Trägerelements
- o: Schälwinkel
- F: Kraft

## Patentansprüche

1. Verbindungssystem (10) mit einem ersten Haftmittel (12), welches eine Mehrzahl von Haftelementen (18, 29, 30, 34, 38, 42) aufweist, und einem Trägerelement (14), wobei das erste Haftmittel (12) ein erstes freies Ende (20) aufweist und an einem, vom ersten freien Ende (20) beabstandeten, Anbindungsbereich (16) schwenkbar an dem Trägerelement (14) angeordnet oder ausgebildet ist, wobei ein erster schwenkbarer Teil (24) des ersten Haftmittels (12) gegenüber dem Trägerelement (14) beweglich ist und wobei der erste schwenkbare Teil (24) eine Länge L vom ersten freien Ende (20) des ersten Haftmittels (12) bis zum Beginn des Anbindungsbereichs (16) aufweist, **dadurch gekennzeichnet,**
**dass** in einem Zustand, in dem der erste schwenkbare Teil (24) gegenüber dem Anbindungsbereich (16) nicht abgewinkelt ist, freie Kanten (11, 13, 15) des ersten schwenkbaren Teils (24) nicht unmittelbar von den Haftelementen (18, 29, 30, 34, 38, 42) des ersten Haftmittels (12) umgeben sind, und dass der erste schwenkbare Teil (24) eine größere Anzahl der Haftelemente (18, 29, 30, 34, 38, 42) aufweist als der Anbindungsbereich (16).

2. Verbindungssystem (10) nach Anspruch 1, wobei das erste Haftmittel (12) ein, dem ersten freien Ende (20) gegenüberliegendes, zweites freies Ende (21) aufweist, wobei sich der Anbindungsbereich (16) zwischen dem ersten freien Ende (20) und dem zweiten freien Ende (21) befindet, wobei das erste Haftmittel (12) einen zweiten schwenkbaren Teil (25) aufweist.

3. Verbindungssystem (10) nach einem der vorhergehenden Ansprüche, wobei das Verhältnis der Länge L des ersten schwenkbaren Teils (24) des ersten Haftmittels (12) und einer Breite B des ersten Haftmittels (12), deren Ausdehnung senkrecht zu L ist, zwischen 0,7:1 und 4:1 liegt.

4. Verbindungssystem (10) nach einem der vorhergehenden Ansprüche, wobei das erste Haftmittel (12) im Anbindungsbereich (16) mittels eines Klebstoffes (26) oder mittels eines thermischen Fügeverfahrens an dem Trägerelement (14) angeordnet ist.

5. Verbindungssystem (10) nach einem der vorhergehenden Ansprüche, wobei die Haftelemente (18, 29, 30, 34, 38, 42) des ersten Haftmittels (12)
a) Hakenelemente (38) sind; und/oder
b) pilzförmige Haftelemente (18) oder palmenförmige Haftelemente (34) oder hyperboloidförmige Haftelemente (34) sind; und/oder
c) Fasern (29) und/oder Schlaufen (30) eines Flauschbandes (32) oder eines Veloursbandes (40) oder eines Gewebes (36) oder eines Vliesstoffes (31) sind; und/oder
d) Nano- und/oder Mikrostrukturen (42) zur Nutzbarmachung intermolekularer Kräfte sind.

6. Verbindungssystem (10) nach einem der vorhergehenden Ansprüche, wobei das erste Haftmittel (12) und/oder das Trägerelement (14) Kunststoff aufweist/aufweisen, insbesondere Polypropylen, Polyamid, Polyethylen, Polyurethan, Polysiloxan und/oder ein thermoplastisches Elastomer.

7. Verbindungssystem (10) nach Anspruch 6, wobei das erste Haftmittel (12) und/oder das Trägerelement (14) ein biologisch abbaubares Kunststoffmaterial und/oder ein biobasiertes Kunststoffmaterial aufweist/aufweisen, insbesondere Polycaprolacton, Polybutyrat, Polybutylensuccinat, Celluloseacetat, biobasiertes Polyethylen, biobasiertes Polypropylen, Polyethylenfuranoat, biobasiertes Polyamid, biobasiertes Polyethylenterephthalat, Polylactid, stärkebasierten Kunststoff und/oder Polyhydroxalkanoat.

8. Verbindungssystem (10) nach einem der vorhergehenden Ansprüche, wobei das Trägerelement (14) ein freies Ende (22) aufweist, wobei eine Länge T des Bereiches des Trägerelements (14) von diesem freien Ende (22) bis zum Beginn des Anbindungsbereichs (16) maximal 500 % der Länge L beträgt, falls T größer L gewählt wird, oder die Länge L maximal 500 % der Länge T beträgt, falls L größer T gewählt wird.

9. Verbindungssystem (10) nach einem der vorhergehenden Ansprüche, wobei die Gesamtheit der Haftelemente (18, 29, 30, 34, 38, 42) des ersten Haftmittels (12) am ersten schwenkbaren Teil (24) des ersten Haftmittels (12) und/oder am zweiten schwenkbaren Teil (25) des ersten Haftmittels (12) gemäß Anspruch 2 angeordnet oder ausgebildet ist.

10. Verbindungssystem (10) nach einem der Ansprüche 1-9, wobei ein weiteres erstes Haftmittel (12') an dem Trägerelement (14) angeordnet oder ausgebildet ist, wobei das weitere erste Haftmittel (12') in einer Ebene des Trägerelements (14) gegenüber dem ersten Haftmittel (12) um 180° gedreht ist, wobei der Anbindungsbereich (16) des ersten Haftmittels (12) und ein weiterer Anbindungsbereich (16') des weiteren ersten Haftmittels (12') näher zueinander liegen als das erste freie Ende (20) des ersten Haftmittels (12) und ein weiteres erstes freies Ende (20') des weiteren ersten Haftmittels (12').

11. Verbindungssystem (10) nach einem der vorhergehenden Ansprüche, wobei das Verbindungssystem (10) ein zweites Haftmittel (28) zum Herstellen einer Haftverbindung mit dem ersten Haftmittel (12) und/oder dem weiteren ersten Haftmittel (12') gemäß Anspruch 10 aufweist, wobei das zweite Haftmittel (28)
a) Hakenelemente (38) aufweist; und/oder
b) pilzförmige Haftelemente (18) oder palmenförmige Haftelemente (34) oder hyperboloidförmige Haftelemente (34) aufweist; und/oder
c) Fasern (29) und/oder Schlaufen (30) eines Flauschbandes (32) oder eines Veloursbandes (40) oder eines Gewebes (36) oder eines Vliesstoffes (31) aufweist; und/oder
d) eine hinreichend glatte Fläche (44) zum Zusammenwirken mit den Mikro- und/oder Nanostrukturen (42) des ersten Haftmittels (12) und/oder des weiteren ersten Haftmittels (12') aufweist.

12. Hygieneartikel (46), insbesondere Windel, welche(r) ein Verbindungssystem (10) nach einem der vorhergehenden Ansprüche aufweist, wobei eine Verschlusslasche (48) des Hygieneartikels (46) das Trägerelement (14) aufweist.

13. Verfahren (100) zum Lösen einer Haftverbindung mit einem Verbindungssystem (10) nach Anspruch 11, umfassend:
i. Ziehen des Trägerelements (14) in eine Richtung weg von einem angebundenen Ende (50) des Trägerelements (14); oder
Ziehen am Trägerelement (14) quer zu einer Richtung von dem angebundenen Ende (50) des Trägerelements (14) zu dem freien Ende (22) des Trägerelements (14); wobei das Trennen des ersten Haftmittels (12) und/oder des weiteren ersten Haftmittels (12') vom zweiten Haftmittel (28) erfolgt.

## Claims

1. A connection system (10) comprising a first adhesive means (12) having a plurality of adhesive elements (18, 29, 30, 34, 38, 42), and a carrier element (14), wherein the first adhesive means (12) has a first free end (20) and is pivotably mounted or formed on the carrier element (14) at a connection region (16) spaced apart from the first free end (20), wherein a first pivotable part (24) of the first adhesive means (12) is movable relative to the carrier element (14), and wherein the first pivotable part (24) has a length L from the first free end (20) of the first adhesive means (12) to the start of the connection region (16), **characterized in that**,
in a state in which the first pivotable part (24) is not angled relative to the connection region (16), free edges (11, 13, 15) of the first pivotable part (24) are not immediately surrounded by the adhesive elements (18, 29, 30, 34, 38, 42) of the first adhesive means (12), and that the first pivotable part (24) comprises a greater number of adhesive elements (18, 29, 30, 34, 38, 42) than the connection region (16).

2. A connection system (10) according to claim 1, wherein the first adhesive means (12) comprises a second free end (21) opposite the first free end (20), wherein the connection region (16) is located between the first free end (20) and the second free end (21), wherein the first adhesive means (12) comprises a second pivotable part (25).

3. A connection system (10) according to any one of the preceding claims, wherein the ratio of the length L of the first pivotable part (24) of the first adhesive means (12) to a width B of the first adhesive means (12), which extends perpendicular to L, is between 0.7:1 and 4:1.

4. A connection system (10) according to any one of the preceding claims, wherein the first adhesive means (12) is arranged on the carrier element (14) in the connection region (16) by means of an adhesive (26) or by means of a thermal joining process.

5. A connection system (10) according to any one of the preceding claims, wherein the adhesive elements (18, 29, 30, 34, 38, 42) of the first adhesive means (12)
a) are hook elements (38); and/or
b) are mushroom-shaped adhesive elements (18) or palm-shaped adhesive elements (34) or hyperboloid-shaped adhesive elements (34); and/or
c) are fibers (29) and/or loops (30) of a loop tape (32) or a velour tape (40) or a woven fabric (36) or a nonwoven fabric (31); and/or
d) are nano- and/or microstructures (42) for utilizing intermolecular forces.

6. A connection system (10) according to any one of the preceding claims, wherein the first adhesive means (12) and/or the carrier element (14) comprise(s) plastic material, in particular polypropylene, polyamide, polyethylene, polyurethane, polysiloxane, and/or a thermoplastic elastomer.

7. A connection system (10) according to claim 6, wherein the first adhesive means (12) and/or the carrier element (14) comprise(s) a biodegradable plastic material and/or a bio-based plastic material, in particular polycaprolactone, polybutyrate, polybutylene succinate, cellulose acetate, bio-based polyethylene, bio-based polypropylene, polyethylene furanoate, bio-based polyamide, bio-based polyethylene terephthalate, polylactide, starch-based plastic material, and/or polyhydroxyalkanoate.

8. A connection system (10) according to any one of the preceding claims, wherein the carrier element (14) has a free end (22), wherein a length T of the region of the carrier element (14) from this free end (22) to the beginning of the connection region (16) is at most 500% of the length L, if T is chosen to be greater than L, or the length L is at most 500% of the length T if L is chosen to be greater than T.

9. A connection system (10) according to any one of the preceding claims, wherein the entirety of the adhesive elements (18, 29, 30, 34, 38, 42) of the first adhesive means (12) are arranged or formed on the first pivotable part (24) of the first adhesive means (12) and/or on the second pivotable part (25) of the first adhesive means (12) according to claim 2.

10. A connection system (10) according to any one of claims 1-9, wherein a further first adhesive means (12') is arranged or formed on the carrier element (14), wherein the further first adhesive means (12') is rotated by 180° relative to the first adhesive means (12) in a plane of the carrier element (14), wherein the connection region (16) of the first adhesive means (12) and a further connection region (16') of the further first adhesive means (12') are closer to each other than the first free end (20) of the first adhesive means (12) and a further first free end (20') of the further first adhesive means (12').

11. A connection system (10) according to any one of the preceding claims, wherein the connection system (10) comprises a second adhesive means (28) for forming an adhesive connection with the first adhesive means (12) and/or the further first adhesive means (12') according to claim 10, wherein the second adhesive means (28)
a) comprises hook elements (38); and/or
b) comprises mushroom-shaped adhesive elements (18) or palm-shaped adhesive elements (34) or hyperboloid-shaped adhesive elements (34); and/or
c) comprises fibers (29) and/or loops (30) of a loop tape (32) or a velour tape (40) or a woven fabric (36) or a nonwoven fabric (31); and/or
d) comprises a sufficiently smooth surface (44) for interacting with the micro- and/or nanostructures (42) of the first adhesive means (12) and/or the further first adhesive means (12').

12. A hygiene product (46), in particular a diaper, comprising a connection system (10) according to any one of the preceding claims, wherein a closure flap (48) of the hygiene product (46) comprises the carrier element (14).

13. A method (100) for releasing an adhesive connection with a connection system (10) according to claim 11, comprising:
i. pulling the carrier element (14) in a direction away from a connected end (50) of the carrier element (14); or
pulling the carrier element (14) transversely to a direction from the connected end (50) of the carrier element (14) to the free end (22) of the carrier element (14); wherein the first adhesive means (12) and/or the further first adhesive means (12') are separated from the second adhesive means (28).

## Revendications

1. Système de liaison (10) avec un premier moyen d'adhérence (12), lequel comprend une pluralité d'éléments d'adhérence (18, 29, 30, 34, 38, 42), et un élément support (14), le premier moyen d'adhérence (12) comprend une première extrémité libre (20) et est disposé ou formé pivotant sur l'élément support (14) dans une zone de liaison (16) espacée de la première extrémité libre (20),une premier partie pivotante (24) du premier moyen d'adhérence (12) étant mobile par rapport à l'élément support (14), et la première partie pivotante (24) comprenant une longueur L depuis la première extrémité libre (20) du premier moyen d'adhérence (12) jusqu'au début de la zone de liaison (16), **caractérisé en ce que**
dans un état où la première partie pivotante (24) n'est pas inclinée par rapport à la zone de liaison (16), les bords libres (11, 13, 15) de la première partie pivotante (24) ne sont pas immédiatement entourés par les éléments d'adhérence (18, 29, 30, 34, 38, 42) du premier moyen d'adhérence (12), et la première partie pivotante (24) comprenant un plus grand nombre d'éléments d'adhérence (18, 29, 30, 34, 38, 42) que la zone de liaison (16).

2. Système de liaison (10) selon la revendication 1, dans lequel le premier moyen d'adhérence (12) comprend une deuxième extrémité libre (21) opposée à la première extrémité libre (20), la zone de liaison (16) étant située entre la première extrémité libre (20) et la deuxième extrémité libre (21), le premier moyen d'adhérence (12) comprenant une deuxième partie pivotante (25).

3. Système de liaison (10) selon l'une des revendications précédentes, dans lequel le rapport entre la longueur L de la première partie pivotante (24) du premier moyen d'adhérence (12) et une largeur B du premier moyen d'adhérence (12), dont l'extension est perpendiculaire à L, est compris entre 0,7:1 et 4:1.

4. Système de liaison (10) selon l'une des revendications précédentes, dans lequel le premier moyen d'adhérence (12) est disposé sur la zone de liaison (16) au moyen d'un moyen d'adhérence (26) ou au moyen d'un procédé d'assemblage thermique sur l'élément support (14).

5. Système de liaison (10) selon l'une des revendications précédentes, dans lequel les éléments d'adhérence (18, 29, 30, 34, 38, 42) du premier moyen d'adhérence (12)
a) sont des éléments crochet (38) ; et/ou
b) sont des éléments d'adhérence en forme de champignon (18) ou des éléments d'adhérence en forme de palmier (34) ou des éléments d'adhérence en forme d'hyperboloïde (34) ; et/ou
c) sont des fibres (29) et/ou des boucles (30) d'une bande bouclée (32) ou d'une bande velours (40) ou d'un tissu (36) ou d'un non-tissé (31) ; et/ou
d) sont des nano- et/ou microstructures (42) pour exploiter des forces intermoléculaires.

6. Système de liaison (10) selon l'une des revendications précédentes, dans lequel le premier moyen d'adhérence (12) et/ou l'élément support (14) comprend de la matière plastique, notamment du polypropylène, du polyamide, du polyéthylène, du polyuréthane, du polysiloxane et/ou un élastomère thermoplastique.

7. Système de liaison (10) selon la revendication 6, dans lequel le premier moyen d'adhérence (12) et/ou l'élément support (14) comprend une matière plastique biodégradable et/ou une matière plastique biosourcée, notamment du polycaprolactone, du polybutyrate, du polybutylène succinate, de l'acétate de cellulose, du polyéthylène biosourcé, du polypropylène biosourcé, du polyéthylène furanoate, du polyamide biosourcé, du polyéthylène téréphtalate biosourcé, du polylactide, de la matière plastique à base d'amidon et/ou du polyhydroxyalcanoate.

8. Système de liaison (10) selon l'une des revendications précédentes, dans lequel l'élément support (14) comprend une extrémité libre (22), une longueur T de la zone de l'élément support (14) depuis cette extrémité libre (22) jusqu'au début de la zone de liaison (16) étant au maximum 500 % de la longueur L, si T est choisie supérieure à L, ou la longueur L étant au maximum 500 % de la longueur T, si L est choisie supérieure à T.

9. Système de liaison (10) selon l'une des revendications précédentes, dans lequel la totalité des éléments d'adhérence (18, 29, 30, 34, 38, 42) du premier moyen d'adhérence (12) est disposée ou formée sur la première partie pivotante (24) du premier moyen d'adhérence (12) et/ou sur la deuxième partie pivotante (25) du premier moyen d'adhérence (12) selon la revendication 2.

10. Système de liaison (10) selon l'une des revendications 1-9, dans lequel un autre premier moyen d'adhérence (12') est disposé ou formé sur l'élément support (14), ledit autre premier moyen d'adhérence (12') étant tourné de 180° dans un plan de l'élément support (14) par rapport au premier moyen d'adhérence (12), la zone de liaison (16) du premier moyen d'adhérence (12) et une autre zone de liaison (16') de l' autre premier moyen d'adhérence (12') étant plus proches l'une de l'autre que la première extrémité libre (20) du premier moyen d'adhérence (12) et une autre première extrémité libre (20') de l'autre premier moyen d'adhérence (12').

11. Système de liaison (10) selon l'une des revendications précédentes, dans lequel le système de liaison (10) comprend un deuxième moyen d'adhérence (28) pour établir une liaison adhésive avec le premier moyen d'adhérence (12) et/ou l'autre premier moyen d'adhérence (12') selon la revendication 10,où le deuxième moyen d'adhérence (28)
a) comprend des éléments crochet (38) ; et/ou
b) comprend des éléments d'adhérence en forme de champignon (18) ou des éléments d'adhérence en forme de palmier (34) ou des éléments d'adhérence en forme d'hyperboloïde (34); et/ou
c) comprend des fibres (29) et/ou des boucles (30) d'une bande bouclée (32) ou d'une bande velours (40) ou d'un tissu (36) ou d'un non-tissé (31) ; et/ou
d) comprend une surface suffisamment lisse (44) pour coopérer avec les micro- et/ou nanostructures (42) du premier moyen d'adhérence (12) et/ou de l'autre premier moyen d'adhérence (12').

12. Article d'hygiène (46), notamment couche, comprenant un système de liaison (10) selon l'une des revendications précédentes, une patte de fermeture (48) de l'article d'hygiène (46) comprenant l'élément support (14).

13. Procédé (100) pour défaire une liaison adhésive avec un système de liaison (10) selon la revendication 11, comprenant :
i. la traction de l'élément de support (14) dans une direction s'éloignant d'une extrémité fixée (50) de l'élément support (14) ; ou
la traction de l'élément de support (14) perpendiculairement à une direction allant de l'extrémité fixée (50) de l'élément de support (14) vers l'extrémité libre (22) de l'élément de support (14) ; ce qui entraîne la séparation du premier adhésif (12) et/ou de l'autre premier adhésif (12') du deuxième adhésif (28).
